(19) 

Europäisches
Patentamt

European
Patent Office

Office européen
des brevets

(11)  **EP 4 470 387 A1**

(12)  **EUROPEAN PATENT APPLICATION**
published in accordance with Art. 153(4) EPC

(43) Date of publication:
**04.12.2024  Bulletin 2024/49**

(21) Application number: **23747042.2**

(22) Date of filing: **26.01.2023**

(51) International Patent Classification (IPC):
*A23J 3/16* (2006.01)    *A23J 3/34* (2006.01)
*A23L 15/00* (2016.01)    *C12N 9/14* (2006.01)

(52) Cooperative Patent Classification (CPC):
**A23J 3/16; A23J 3/34; A23L 15/00; C12N 9/14**

(86) International application number:
**PCT/JP2023/002474**

(87) International publication number:
**WO 2023/145832 (03.08.2023 Gazette 2023/31)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.01.2022  JP 2022011269**

(71) Applicant: **Amano Enzyme Inc.
Nagoya-shi
Aichi 460-8630 (JP)**

(72) Inventors:
• **HATTA, Hajime
Kyoto-shi, Kyoto 605-8501 (JP)**
• **KOZUKA, Misa
Kakamigahara-shi, Gifu 509-0109 (JP)**

(74) Representative: **Müller-Boré & Partner
Patentanwälte PartG mbB
Friedenheimer Brücke 21
80639 München (DE)**

(54) **PROPERTY MODIFIER FOR HEAT-COAGULATED GEL OF EGG WHITE PROTEIN**

(57)  The purpose of the present invention is to provide a novel technology for modifying the properties of the heat-coagulated gel of egg white protein. In the present invention, a composition containing soy protein that has been subjected to protein deamidation is capable of improving the hardness, resiliency, and/or water-holding capacity of the heat-coagulated gel of egg white protein.

EP 4 470 387 A1

## Description

Technical Field

The present invention relates to a physical property modifier for a heat-coagulated gel of egg white protein. More specifically, the present invention relates to a physical property modifier that improves the hardness, elasticity, and/or water-holding capacity of a heat-coagulated gel of egg white protein.

Background Art

The egg white protein has characteristics of forming a gel network by heat coagulation, and the characteristics are widely used in food production. For example, it is known that a heat-coagulated gel of egg white protein is added to food for the purpose of improving a binding property and/or texture characteristics. In addition, techniques for modifying characteristics such as hardness and/or elasticity of the heat-coagulated gel of such egg white protein have also been studied. For example, NPL 1 discloses that the jelly strength is increased by adding salt in a study on coagulation characteristics of eggs. PTL 1 shows that when an egg processed food such as a boiled egg is immersed in a solution containing transglutaminase, hardness and elasticity can be imparted as compared with the case where the processed food is not treated with transglutaminase.

Citation List

Non Patent Literature

NPL 1: Journal of cookery science of Japan Vol. 15 No. 2 (1982) p. 114 to 118

Patent Literature

PTL 1: Japanese Patent Laid-open Publication No. 2017-175976

Summary of Invention

Technical Problem

Conventionally known techniques for modifying characteristics such as hardness and/or elasticity of a heat-coagulated gel of egg white protein are accompanied by various limitations. For example, the technique of adding salt has limited application to foods corresponding to a low salt intention. In addition, the technique using transglutaminase is limited in the production process in that the egg white protein is heat-coagulated in the heating inactivation process of the enzyme.

Therefore, an object of the present invention is to provide a new technique for modifying the physical properties of the heat-coagulated gel of egg white protein without these limitations.

Solution to Problem

As a result of intensive studies, the present inventor has found that a composition containing a soybean protein that has been previously protein-deamidated can improve hardness, elasticity, and/or water-holding capacity of egg white protein. The present invention has been completed by further conducting studies based on this finding. That is, the present invention provides the inventions of the following aspects.

Item 1. A physical property modifier for a heat-coagulated gel of egg white protein, including a composition containing a protein-deamidated soybean protein, in which
the physical property modification is improvement of hardness, elasticity, and/or water-holding capacity.

Item 2. A method for producing a physical property modifier for a heat-coagulated gel of egg white protein, including an enzyme treatment step of treating a soybean protein-containing composition with a protein deamidase, in which
the physical property modification is improvement of hardness, elasticity, and/or water-holding capacity.

Item 3. The method according to item 2, in which the protein deamidase is protein glutaminase.

Item 4. The method according to item 2 or 3, further including a high-temperature heat treatment step of performing heat treatment at 75°C or higher after the enzyme treatment step.

Item 5. A heat-coagulated gel including: a composition containing a protein-deamidated soybean protein; and egg white protein.

Item 6. A method for producing a heat-coagulated gel, including:

a mixing step of mixing a composition containing a protein-deamidated soybean protein with egg white protein to prepare an egg white protein mixture; and
a heat coagulation step of heat-coagulating the egg white protein mixture.

Item 7. A food including the heat-coagulated gel according to item 5.

Item 8. An enzyme preparation which includes a protein deamidase, and is used in the method according to any one of items 2 to 4.

Item 9. The enzyme preparation according to item 8, in which the protein deamidase is protein glutaminase.

Item 10. A method for modifying physical properties of a heat-coagulated gel of egg white protein, including:

a mixing step of mixing a composition containing a protein-deamidated soybean protein with egg white protein to prepare an egg white protein mixture; and
a heat coagulation step of heat-coagulating the egg white protein mixture, in which
the physical property modification is improvement of hardness, elasticity, and/or water-holding capacity.

Item 11. A use of a composition containing a protein-deamidated soybean protein for modifying physical properties of a heat-coagulated gel of egg white protein, in which
the physical property modification is improvement of hardness, elasticity, and/or water-holding capacity.

Advantageous Effects of Invention

According to the present invention, a new technique for modifying the physical properties of a heat-coagulated gel of egg white protein is provided.

Description of Embodiments

1. Physical property modifier for heat-coagulated gel of egg white protein

The physical property modifier for a heat-coagulated gel of egg white protein of the present invention includes a composition containing a protein-deamidated soybean protein, and is used for improving hardness, elasticity, and/or water-holding capacity of the heat-coagulated gel of egg white protein.

1-1. Active ingredients

The composition containing a protein-deamidated soybean protein is contained as an active ingredient of the physical property modifier for a heat-coagulated gel of egg white protein of the present invention. A specific aspect of the protein-deamidated soybean protein is a natural soybean protein whose amide group-containing side chain is decomposed without cleaving of a peptide bond and crosslinking of the protein.

The protein deamidation rate of the composition containing a protein-deamidated soybean protein is not particularly limited as long as the effect of the present invention is exhibited. The protein deamidation rate is, for example, 5 $\mu$mol or more per 1 g of soybean protein in terms of the production amount of ammonia released by deamidation. From the viewpoint of further improving the effect of modifying the physical properties of the heat-coagulated gel of egg white protein, the protein deamidation rate is, for example, preferably 10 $\mu$mol or more, more preferably 15 $\mu$mol or more, still more preferably 20 $\mu$mol or more, still more preferably 25 $\mu$mol or more, and still more preferably 30 $\mu$mol or more, per 1 g of soybean protein in terms of the production amount of ammonia released by deamidation. In addition, in the composition containing a protein-deamidated soybean protein, the upper limit of the protein deamidation rate is not particularly limited. All the amide group-containing side chains of the soybean protein may be deamidated, or the protein deamidation rate may be, for example, 50 $\mu$mol or less, 45 $\mu$mol or less, 40 $\mu$mol or less, or 37 $\mu$mol or less, per 1 g of the soybean protein in terms of the production amount of ammonia released by deamidation.

1-2. Other components

The physical property modifier for a heat-coagulated gel of egg white protein of the present invention may contain other components in addition to the composition containing a protein-deamidated soybean protein as long as the effect of the present invention is not affected. Examples of other components include enzymes, additives, and bases.

Examples of the enzyme include a protein deamidase. More specific examples of the protein deamidase include those used for the preparation of the active ingredient and remaining in the active ingredient. A preferred example of the physical property modifier for a heat-coagulated gel of egg white protein of the present invention does not substantially include a protein deamidase activity. The phrase "does not substantially include a protein deamidase activity" means that an effective amount of an enzyme for protein deamidation is not contained, and more preferably, protein deamidase activity is not included at all.

Examples of the additive and base include excipients, buffers, antioxidants, ultraviolet inhibitors, preservatives, antiseptics, pH adjusters, dispersants, emulsifiers, solubilizing agents, solvents (particularly water and the like), vitamins, minerals, and seasonings (sweeteners and the like). These additives and bases may be used alone or in combination of two or more thereof. In addition, the contents of these additives and bases may be appropriately set according to the type of these components and/or the preparation form of the physical property modifier for a heat-coagulated gel of egg white protein.

1-3. Form

The form of the physical property modifier for a heat-coagulated gel of egg white protein of the present invention is not particularly limited. Examples thereof include dry preparations in powdery form, fine particulate form, and granular form, and liquid preparations.

1-4. Application

The physical property modifier for a heat-coagulated gel of egg white protein of the present invention is used for improving hardness, elasticity, and/or water-holding capacity of the heat-coagulated gel of egg white protein. Hereinafter, in the present specification, the "modification of physical properties of the heat-coagulated gel of egg white protein" means improvement of hardness, elasticity, and/or water-holding capacity of the heat-coagulated gel of egg white protein. More specifically, the phrase "the hardness, the elasticity, and the water-holding capacity are respectively improved" means that these physical property values are increased. Preferably, the physical property modifier for a heat-coagulated gel of egg white protein of the present invention is used for improving at least hardness, more preferably hardness and elasticity, and still more preferably hardness, elasticity, and water-holding capacity of the heat-coagulated gel of egg white protein.

2. Method for producing physical property modifier for heat-coagulated gel of egg white protein

The method for producing a physical property modifier for a heat-coagulated gel of egg white protein of the present invention is a method for producing the physical property modifier for a heat-coagulated gel of egg white protein described in the above "1. Physical property modifier for heat-coagulated gel of egg white protein", and includes an enzyme treatment step of treating a soybean protein-containing composition with a protein deamidase.

2-1. Enzyme treatment step

In the enzyme treatment step, the soybean protein-containing composition is treated with a protein deamidase.

2-1-1. Soybean protein-containing composition

The soybean protein-containing composition is not particularly limited as long as it contains a soybean protein. The content of the soybean protein is not particularly limited, and is, for example, 0.1 to 20 wt% or 0.5 to 15 wt%, preferably 1 to 10 wt% or 1.5 to 7.5 wt%, more preferably 2 to 5 wt%, and still more preferably 3 to 4 wt%.

The soybean protein-containing composition may contain components other than soybean protein. Examples of the component other than soybean protein include components derived from soybean other than protein, and components not derived from soybean. Examples of the component derived from soybean other than protein include carbohydrate, dietary fiber, lipid, saponin, lecithin, isoflavone, and fatty acid. Examples of the component not derived from soybean include additives and/or bases such as sugar, salts, fats and oils, calcium lactate, spices, fragrances, excipients, buffers, antioxidants, ultraviolet inhibitors, preservatives, antiseptics, pH adjusters, dispersants, emulsifiers, solubilizing agents, thickeners (starch and the like), stabilizers, and solvents (particularly water and the like). The soybean protein-containing composition may contain one of these components other than the soybean protein alone or in combination of two or more thereof.

In the enzyme treatment step, the soybean protein-containing composition is used in the form of a liquid.

Specific examples of the soybean protein-containing composition include soy milk, a soybean beverage, a soybean protein beverage, a soybean liquid, and a soy pulp liquid. Examples of the soy milk include plain soy milk and prepared soy milk. Examples of the soybean beverage include soy milk mixed beverages having a soybean solid content of 2 wt% or more, preferably 4 wt% or more. Examples of the soybean protein beverage include beverages containing soybean protein concentrated or purified from soybean as a main component. Examples of the soybean liquid include liquids obtained by suspending, dissolving and/or homogenizing a powder of dehulled soybean and/or whole soybean in water. Examples of the soy pulp liquid include liquids obtained by dissolving, suspending and/or homogenizing soy pulp by a known method. In the present invention, these soybean protein-containing compositions may be used alone or in combination of two or more thereof.

Among these soybean protein-containing compositions, soy milk, a soybean beverage, a soybean protein beverage, and a soybean liquid are preferable, soy milk is more preferable, and plain soy milk is particularly preferable, from the viewpoint of further improving the effect of modifying the physical properties of the heat-coagulated gel of egg white protein.

2-1-2. Protein deamidase

The type, origin, and the like of the protein deamidase are not particularly limited as long as the enzyme exhibits an action of decomposing an amide group-containing side chain of a protein without cleaving of a peptide bond and crosslinking of the protein. Examples of the protein deamidase include protein deamidase derived from the genus Chryseobacterium, Flavobacterium, Empedobacter, Sphingobacterium, Aureobacterium, or Myroides, and protein glutaminase derived from the genus Chryseobacterium, which are disclosed in JP 2000-50887 A, JP 2001-218590 A, and WO 2006/075772 A. These protein deamidase may be used alone or in combination of two or more thereof.

Examples of the protein deamidase include protein glutaminase and protein asparaginase, and in a broad sense, also includes protein arginine deiminase. Preferable examples of the protein deamidase include protein glutaminase.

Among these protein deamidases, protein glutaminase is preferable, protein deamidase derived from the genus Chryseobacterium is more preferable, protein glutaminase derived from the genus Chryseobacterium is still more preferable, and protein glutaminase derived from Chryseobacterium proteolyticum species is still even more preferable from the viewpoint of further improving the effect of modifying the physical properties of the heat-coagulated gel of the egg white protein.

The protein deamidase can be prepared from a culture solution of a microorganism from which the protein deamidase is derived. Specific examples of the preparation method include a method of recovering a protein deamidase from a culture solution or a bacterial cell of the above-described microorganism. For example, in the case of using a microorganism that secretes protein deamidase, bacterial cells are collected from the culture solution in advance by filtration, centrifugation or the like as necessary, and then the enzyme can be separated and/or purified. In addition, in the case of using a microorganism that does not secrete protein deamidase, bacterial cells are recovered from the culture solution in advance as necessary, and then the bacterial cells are disrupted by pressurization treatment, ultrasonic treatment, or the like to expose the enzyme, and then the enzyme can be separated and/or purified. As the method for separating and/or purifying an enzyme, a known method for separating and/or purifying a protein can be used without particular limitation. Examples of the method include a centrifugation method, a UF concentration method, a salting-out method, and various chromatography methods using an ion exchange resin or the like. The separated and/or purified enzyme can be powdered by a drying method such as freeze-drying or drying under reduced pressure.

As the protein deamidase, a protein deamidase in the form of an enzyme preparation formulated by powdering using an appropriate excipient and/or drying aid in the drying method can be used. As the protein deamidase, a protein deamidase in the form of an enzyme preparation formulated as a liquid product by adding an appropriate additive to the separated and/or purified enzyme and subjecting the enzyme to filtration sterilization can also be used.

The amount of protein deamidase used is not particularly limited, but the amount of protein deamidase used per 1 g of the soybean protein-containing composition is, for example, 0.01 U or more, 0.04 U or more, or 0.08 U or more. From the viewpoint of further improving the effect of modifying the physical properties of the heat-coagulated gel of egg white protein, the amount of protein deamidase used per 1 g of the soybean protein-containing composition is, for example, preferably 0.1 U or more, more preferably 0.15 U or more, still more preferably 0.2 U or more, or 0.25 U or more, still more preferably 0.3 U or more, or 0.34 U or more. The upper limit of the range of the amount of protein deamidase used per 1 g of the soybean protein-containing composition is not particularly limited, and is for example, 30 U or less, 25 U or less, 15 U or less, 10 U or less, 5 U or less, 4 U or less, or 3.8 U or less.

The amount of protein deamidase used is not particularly limited, but the amount of protein deamidase used per 1 g of the soybean protein contained in the soybean protein-containing composition is, for example, 0.1 U or more, 0.5 U or more, or 1 U or more. The amount of protein deamidase used per 1 g of the soybean protein contained in the soybean protein-containing composition is, for example, preferably 2 U or more, more preferably 4 U or more, still more preferably 6 U or more, or 8 U or more, still even more preferably 9 U or more, or 9.5 U or more, from the viewpoint of further improving the effect of modifying the physical properties of the heat-coagulated gel of egg white protein. The upper limit of the range of the amount of protein deamidase used per 1 g of the soybean protein contained in the soybean protein-containing composition is not particularly limited, and is, for example, 1,000 U or less, 500 U or less, 200 U or less, 150 U or less, 120 U or less, or 110 U or less.

For the activity of the protein deamidase, benzyloxycarbonyl-L-glutaminylglycine (Z-Gln-Gly) is used as a substrate, and the amount of enzyme releasing 1 μmol of ammonia per minute is defined as 1 unit (1 U).

2-1-3. Enzyme treatment conditions

As specific conditions (for example, temperature, pH, time) for treating the soybean protein-containing composition with the protein deamidase in the enzyme treatment step, conditions under which the protein deamidase can effectively deamidate the soybean protein in the soybean protein-containing composition can be appropriately selected.

The reaction temperature may be set based on the optimum temperature or the like of the protein deamidase to be used as long as the protein deamidase to be used is not inactivated by heat, and is, for example, 20 to 70°C, preferably 40 to 60°C, more preferably 45 to 55°C.

The pH may be set based on the optimum pH or the like of the protein deamidase to be used, and is, for example, 5 to 10, preferably 5 to 8, more preferably 6 to 7.5 at a pH of a mixture containing the soybean protein-containing composition and the protein deamidase at 25°C.

The reaction time may be set based on the scale of the mixture containing the soybean protein-containing composition and the protein deamidase, and is, for example, 15 minutes to 24 hours, preferably 20 minutes to 12 hours, more preferably 30 minutes to 6 hours, 40 minutes to 3 hours, or 50 minutes to 90 minutes.

2-2. High-temperature heat treatment step

In the present invention, from the viewpoint of further improving the effect of modifying the physical properties of the heat-coagulated gel of egg white protein, it is preferable to further include a high-temperature heat treatment step of performing heat treatment at 75°C or higher after the enzyme treatment step.

The more preferable temperature in the high-temperature heat treatment step is, for example, preferably 78°C or higher, more preferably 85°C or higher, still more preferably 90°C or higher, still even more preferably 94°C or higher, and particularly preferably 98°C or higher from the viewpoint of further improving the effect of modifying the physical properties of the heat-coagulated gel of egg white protein. The upper limit of the temperature in the high-temperature heat treatment step is, for example, a boiling temperature of the reaction mixture containing the soybean protein-containing composition and the protein deamidase at normal pressure, or 190°C or lower, 170°C or lower, 150°C or lower, or 130°C or lower under pressure.

The treatment time in the high-temperature heat treatment step is not particularly limited, and is, for example, 5 to 60 minutes, preferably 10 to 50 minutes, more preferably 20 to 40 minutes.

2-3. Other steps

In the present invention, in addition to the above steps, an optional step for formulating the physical property modifier for a heat-coagulated gel of egg white protein can be included. Examples of other steps include a step of blending other components other than the active ingredient, a filtration step, a drying step, and a granulation step. The production method of the present invention can include one of these steps alone or in combination of two or more thereof.

3. Heat-coagulated gel

The composition containing a protein-deamidated soybean protein, which is an active ingredient of the physical property modifier for a heat-coagulated gel of egg white protein described in the above "1. Physical property modifier for heat-coagulated gel of egg white protein" can modify the physical properties of the heat-coagulated gel of egg white protein. Therefore, a heat-coagulated gel of egg white protein with modified physical properties (specifically, hardness, elasticity, and/or water-holding capacity were improved) is obtained by heat-coagulating the egg white protein in the presence of the composition containing a protein-deamidated soybean protein. Accordingly, the present invention also provides a heat-coagulated gel including: a composition containing a protein-deamidated soybean protein; and egg white protein.

The heat-coagulated gel of the present invention includes: a composition containing a protein-deamidated soybean protein; and egg white protein, and the egg white protein is heat-coagulated. In a preferred aspect, the egg white protein is not subjected to protein deamidation.

In the heat-coagulated gel of the present invention, the content of the protein-deamidated soybean protein relative to the egg white protein is not particularly limited, but the content of the protein-deamidated soybean protein per 1 g of the egg white protein is, for example, 0.001 to 2 g or 0.01 to 1.5 g, preferably 0.03 to 1.2 g, 0.05 to 1 g or 0.1 to 0.8 g, more preferably 0.2 to 0.6 g, and further preferably 0.3 to 0.4 g.

The heat-coagulated gel of the present invention has improved hardness, elasticity, and/or water-holding capacity, preferably has improved hardness and elasticity, and more preferably has improved hardness, elasticity, and water-holding capacity, as compared with a heat-coagulated gel of egg white protein obtained by heat-coagulation under the same conditions except that the composition containing a protein-deamidated soybean protein is not used.

4. Method for producing heat-coagulated gel

The method for producing a heat-coagulated gel of the present invention is a method for producing the heat-coagulated gel described in the above "3. Heat-coagulated gel", and includes a mixing step of mixing the composition containing a protein-deamidated soybean protein with egg white protein to prepare an egg white protein mixture, and a heat coagulation step of heat-coagulating the egg white protein mixture.

The egg white protein used in the mixing step is not particularly limited as long as it is egg white protein of a bird's egg, but is, for example, preferably a bird's edible egg, and more preferably egg white protein of a hen's egg. In addition, the egg white protein is not particularly limited as long as it is a protein constituting egg white, and examples thereof include ovalbumin, ovotransferrin, ovomucoid, and ovomucin. These egg white proteins may be used alone or in combination of two or more thereof.

The specific form of the egg white protein used in the mixing step is not particularly limited as long as egg white protein that is not heat-coagulated is contained. Examples of the egg white protein include purified egg white protein, raw egg white (including thick egg white and watery egg white), dried egg white, raw whole egg, and dried whole egg, and mixtures of these with other components (for example, other food ingredients).

The mixing ratio between the composition containing a protein-deamidated soybean protein and the egg white protein is not particularly limited. The mixing ratio is, for example, a ratio at which the content of the protein-deamidated soybean protein per 1 g of the egg white protein is, for example, 0.001 to 2 g or 0.01 to 1.5 g, preferably 0.03 to 1.2 g, 0.05 to 1 g or 0.1 to 0.8 g, more preferably 0.2 to 0.6 g, and still more preferably 0.3 to 0.4 g.

Another example of the mixing ratio between the composition containing a protein-deamidated soybean protein and the egg white protein is a ratio at which the amount of the composition containing a protein-deamidated soybean protein per 1 g of raw egg white (a combination of thick egg white and watery egg white) is, for example, 0.1 to 5 g or 0.2 to 2 g, preferably 0.4 to 1.6 g, more preferably 0.6 to 1.4 g, and still more preferably 0.8 to 1.2 g.

The heat coagulation temperature in the heat coagulation step is not particularly limited, and is, for example, 70°C to 100°C, preferably 78°C to 100°C.

The heating time in the heat coagulation step is not particularly limited, and the required time for obtaining a desired heat coagulation form may be appropriately determined. The heating time is, for example, 5 minutes to 2 hours, or 10 minutes to 1 hour, preferably 15 minutes to 45 minutes.

5. Food containing heat-coagulated gel

The food of the present invention contains the heat-coagulated gel described in the above "3. Heat-coagulated gel". Specifically, the food of the present invention is not particularly limited as long as it is a food obtained by cooking using egg white.

Examples of the food include cooked egg foods, and foods cooked using egg white as a binder. Examples of the cooked egg food include boiled eggs, hot-spring eggs, poached eggs, scrambled eggs, thick omelets, Japanese-style rolled omelets, egg ingredients for Tianjin rice bowl, egg soups, egg-drop, quiches, omelets, thin omelets (egg sheets for omelet rice, thin egg strips), chawanmushi, steamed egg custard, puddings, and cakes. Examples of the food cooked using egg white as a binder include fish pastes such as kamaboko, chikuwa, hanpen, datemaki, and fish sausages; processed meats such as hamburg steaks, meat balls, patties, meat loafs, or minced meat cutlets; and foods obtained by substituting these with plant-derived pseudo meats.

6. Enzyme preparation

As described above, the protein deamidase serves as a material of the physical property modifier for a heat-coagulated gel of egg white protein. Therefore, the present invention also provides an enzyme preparation which contains a protein deamidase and is used in the production method described in the above "2. Method for producing physical property modifier for heat-coagulated gel of egg white protein".

The content of the protein deamidase contained in the enzyme preparation of the present invention can be appropriately determined to such an extent that the protein deamidase can act as an active ingredient when the enzyme preparation of the present invention is used.

Details of the protein deamidase contained in the enzyme preparation of the present invention are as described in the above "2-1-2. Protein deamidase" of "2. Method for producing physical property modifier for heat-coagulated gel of egg white protein".

The enzyme preparation of the present invention may be composed only of protein deamidase, or may contain an additive and/or a base that is/are acceptable in the formulation of the enzyme preparation as a component other than the protein deamidase. Examples of such an additive and a base include excipients, buffers, antioxidants, ultraviolet inhibitors, preservatives, antiseptics, pH adjusters, dispersants, emulsifiers, solubilizing agents, carriers, and solvents (water or the like). These additives and bases may be used alone or in combination of two or more thereof. In addition, the contents of these additives and bases may be appropriately set according to the type and/or preparation form of these components.

The form of the enzyme preparation of the present invention are not particularly limited, and examples thereof include dry preparations in powdery form, fine particulate form, and granular form, and liquid preparations.

Example

Hereinafter, the present invention will be specifically described with reference to Examples, but the present invention is not to be construed as being limited to the following Examples.

Used enzyme

Protein glutaminase derived from Chryseobacterium proteolyticum manufactured by Amano Enzyme Inc. was used as protein deamidase (hereinafter, also referred to as "PG").

The protein deamidase activity value was measured by the following method.

To 1 mL of a 0.2 M phosphate buffer (pH 6.5) containing 30 mM Z-Gln-Gly, 0.1 mL of a sample solution containing protein deamidase was added, and the mixture was allowed to stand at 37°C for 10 minutes, and then 1 mL of a 0.4 M TCA solution was added to stop the reaction. As a blank, 1 mL of a 0.4 M TCA solution was added to 1 mL of a 0.2 M phosphate buffer (pH 6.5) containing 30 mM Z-Gln-Gly, and then 0.1 mL of a sample solution containing protein deamidase was further added, and the mixture was allowed to stand at 37°C for 10 minutes.

The amount of ammonia generated in the reaction liquid was measured for the obtained solution using Ammonia Test Wako (FUJIFILM Wako Pure Chemical Corporation). The ammonia concentration in the reaction liquid was determined from a calibration curve representing the relationship between the ammonia concentration and the absorbance (630 nm) prepared using an ammonia standard liquid (ammonium chloride).

The amount of enzyme that produces 1 $\mu$mol of ammonia per minute was defined as one unit (1 U), and the activity of the protein deamidase was calculated from the following formula. In the formula, the reaction liquid amount is 2.1, the enzyme solution amount is 0.1, and Df is a dilution rate of the enzyme solution. Further, 17.03 is a molecular weight of ammonia.

Protein deamidase activity (U/mL) = ammonia concentration in reaction liquid (mg/L) $\times$ (1/17.03) $\times$ (reaction liquid amount/enzyme solution amount) $\times$ (1/10) $\times$ Df
$\qquad$ [Mathematical Formula 1]

The used protein glutaminase is inactivated by heat treatment at 70°C for 10 minutes. Specifically, when the protein deamidase activity before heating is 100%, the protein deamidase activity after heating under the above conditions is almost 0%.

Used materials

Soy milk (protein 3.6 wt%)
Raw egg white (protein 10 wt%)

Test Example 1

(1) Methods

(1-1) Production of composition containing protein-deamidated soybean protein (physical property modifier for heat-coagulated gel of egg white protein)

Soy milk was preheated to 50°C, 10 U or 100 U of protein glutaminase per 1 g of soybean protein was added, and the mixture was allowed to react at 50°C for 60 minutes, thus obtaining protein-deamidated soy milk.

(1-2) Production of heat-coagulated gel of egg white protein

Thick egg white and watery egg white were mixed and filtered with gauze to obtain an egg white liquid. The egg white liquid preheated at 50°C in advance was mixed with an equal amount (in terms of weight) of the protein-deamidated soy milk, and the pH (25°C) of the mixture was adjusted to 6.7 to obtain an egg white protein mixture. The egg white protein mixture was placed in wells of a 96 well microplate and heated in a thermostat bath at 80°C for 30 minutes to obtain a heat-coagulated gel of Examples.

(2) Measurement

(2-1) Degree of protein deamidation of physical property modifier for heat-coagulated gel of egg white protein

The degree of protein deamidation of the physical property modifier for a heat-coagulated gel of egg white protein was measured as the production amount of ammonia released by treatment with protein glutaminase. Specifically, the amount of ammonia generated in the physical property modifier for a heat-coagulated gel of egg white protein was measured using Ammonia Test Wako (FUJIFILM Wako Pure Chemical Corporation). The production amount of ammonia in the physical property modifier for a heat-coagulated gel of egg white protein was determined from a calibration curve representing the relationship between the ammonia concentration and the absorbance (630 nm) prepared using an ammonia standard liquid (ammonium chloride). The obtained production amount of ammonia was converted into an amount per 1 g of soybean protein subjected to measurement. The results are shown in Table 1.

(2-2)

The following measurement was performed on the obtained heat-coagulated gels. Separately, heat-coagulated gels of Comparative Example obtained by performing the same operation as in the above (1) except that the protein deamidation treatment was not performed were produced, and the same measurement was also performed on each of the heat-coagulated gels of Comparative Example.

(2-2-1) Hardness and elasticity

The hardness and elasticity of the heat-coagulated gel was measured using a compression tester (Texo Graph; manufactured by NIPPON SHOKUHIN KAIHATSU KENKYUSHO KK) and using a cylindrical plunger (cross-sectional area; 0.125 $cm^3$) at a speed of 0.4 mm/sec. The hardness is a physical property value measured as a maximum load (g/$cm^3$) recorded during initial compression. The elasticity is a physical property value expressed by the following formula using the compression work load CW (unit: gf×cm/$cm^2$; representing an area surrounded by a compression curve, a line segment drawn from a vertex of the compression curve to the X axis, and the X axis) and the recovery work load DW (unit: gf×cm/$cm^2$; representing an area surrounded by a recovery curve, a line segment drawn from a vertex of the recovery curve to the Y axis, and the Y axis).

[Mathematical Formula 2]

$$\text{Elasticity (\%)} = (DW/CW) \times 100$$

Furthermore, for any physical property value, a relative value when the physical property value in Comparative Examples was 1 was derived. The results are shown in Table 1.

(2-2-2) Water-holding capacity

On a waste (KIMTOWEL) which had been previously weighed (g) (weight A), a heat-coagulated gel which had been cut to a thickness of 0.5 cm and previously weighed (g: initial weight) was placed, and this was left for 30 minutes. After standing, the heat-coagulated gel was removed from the waste, and the weight (g) of the waste was measured (weight B). The amount obtained by subtracting the weight A from the weight B was taken as the water separation amount (g), and the water-holding capacity was measured according to the following formula. The results are shown in Table 1.

[Mathematical Formula 3]

$$\text{Water-holding capacity (\%)} = \frac{\text{Initial weight (g) - Water separation amount (g)}}{\text{Initial weight (g)}} \times 100$$

(3) Results

[Table 1]

|  | PG used amount (U/g soybean protein) | Production amount of ammonia released by PG treatment (μmol/g soybean protein) | Hardness (relative value) | Elasticity (relative value) | Water-holding capacity (%) |
|---|---|---|---|---|---|
| Comparative Example 1 | 0 |  | 1 | 1 | 87.6 |
| Example 1 | 10 | 23 | 222 | 204 | 91.5 |
| Example 2 | 100 | 34 | 2.51 | 1.88 | NA |

As shown in Table 1, the heat-coagulated gels of the egg white proteins prepared using the protein-deamidated soy milks (Examples 1 and 2) had remarkably improved hardness and elasticity, as compared with the heat-coagulated gel of egg white protein prepared using soy milk not subjected to the protein deamidation treatment (Comparative Example 1). Furthermore, it can be confirmed that the heat-coagulated gel of egg white protein of Example 1 also had improved water-holding capacity, and for the heat-coagulated gel of egg white protein of Example 2, water-holding capacity data was not specifically acquired (NA: not available), but it is presumed that the water-holding capacity was similarly improved as comprehensively judged from the results of Example 1 in Table 1 and Example 3 in Table 2 described later.

Test Example 2

Heat-coagulated gels of Examples and Comparative Examples were produced in the same manner as in Test Example 1 except that the egg white protein mixture was placed in a casing tube and the heating temperature was changed to 100°C (boiling water bath) in the production of the heat-coagulated gel. Each physical property of the obtained heat-coagulated gels was measured in the same manner as in Test Example 1. The results are shown in Table 2.

[Table 2]

|  | PG used amount (U/g soybean protein) | Hardness (relative value) | Elasticity (relative value) | Water-holding capacity (%) |
|---|---|---|---|---|
| Comparative Example 2 | 0 | 1 | 1 | 88.7 |
| Example 3 | 10 | 1.24 | 1.23 | 90.1 |

As shown in Table 2, even when the preparation temperature of the heat-coagulated gel was 100°C, the heat-coagulated gel of egg white protein prepared using protein-deamidated soy milk (Example 3) had improved hardness, elasticity, and water-holding capacity, as compared with the heat-coagulated gel of egg white protein prepared using soy milk not subjected to the protein deamidation treatment (Comparative Example 2).

Test Example 3

Protein glutaminase (10 U/g per 1 g of soybean protein) was added to soy milk preheated to 50°C, and the mixture was allowed to react at 50°C for 60 minutes, then the mixture was further divided into three equal parts, and each of the three equal parts was placed in ice water (4°C), a thermostat bath at 80°C, or a steamer near 100°C, and left to stand for 30 minutes to obtain protein-deamidated soy milks.

Heat-coagulated gels of Examples were obtained in the same manner as in (1-2) in Test Example 1, except that the egg white protein mixture prepared using the protein-deamidated soy milk obtained as described above was placed in a casing tube, and heat-coagulated.

Each physical property of the obtained heat-coagulated gels of Examples and heat-coagulated gel of Comparative Example 1 (additional test was performed using a casing tube) was measured in the same manner as in Test Example 1. The results are shown in Table 3.

[Table 3]

| | Heating condition after PG treatment of soy milk | Hardness (relative value) | Elasticity (relative value) |
|---|---|---|---|
| Comparative Example 1 | - | 1 | 1 |
| Example 4 | 4°C | 1.56 | 1.79 |
| Example 5 | 80°C | 2.5 | 2.16 |
| Example 6 | 100°C | 2.82 | 2.8 |

As shown in Table 3, it has been found that when a heat-coagulated gel of egg white protein is prepared using a protein-deamidated soy milk obtained by subjecting soy milk to protein deamidation treatment and then subjecting the protein-deamidated soy milk to high-temperature heat treatment at 80°C or 100°C (Examples 5 and 6), the hardness and elasticity of the heat-coagulated gel are remarkably improved as compared with the case where a heat-coagulated gel of egg white protein is prepared using a protein-deamidated soy milk not subjected to the high heat treatment (Example 4).

**Claims**

1. A physical property modifier for a heat-coagulated gel of egg white protein, comprising a composition containing a protein-deamidated soybean protein, wherein
the physical property modification is improvement of hardness, elasticity, and/or water-holding capacity.

2. A method for producing a physical property modifier for a heat-coagulated gel of egg white protein, comprising an enzyme treatment step of treating a soybean protein-containing composition with a protein deamidase, wherein
the physical property modification is improvement of hardness, elasticity, and/or water-holding capacity.

3. The method according to claim 2, wherein the protein deamidase is protein glutaminase.

4. The method according to claim 2, further comprising a high-temperature heat treatment step of performing heat treatment at 75°C or higher after the enzyme treatment step.

5. A heat-coagulated gel comprising: a composition containing a protein-deamidated soybean protein; and egg white protein.

6. A method for producing a heat-coagulated gel, comprising:

a mixing step of mixing a composition containing a protein-deamidated soybean protein with egg white protein to prepare an egg white protein mixture; and
a heat coagulation step of heat-coagulating the egg white protein mixture.

7. A food comprising the heat-coagulated gel according to claim 5.

8. An enzyme preparation which comprises a protein deamidase, and is used in the method according to claim 2.

9. The enzyme preparation according to claim 8, wherein the protein deamidase is protein glutaminase.

## INTERNATIONAL SEARCH REPORT

| International application No. |
|---|
| **PCT/JP2023/002474** |

**A. CLASSIFICATION OF SUBJECT MATTER**

*A23J 3/16*(2006.01)i; *A23J 3/34*(2006.01)i; *A23L 15/00*(2016.01)i; *C12N 9/14*(2006.01)i
FI: A23J3/16; A23J3/34; A23L15/00 B; C12N9/14

According to International Patent Classification (IPC) or to both national classification and IPC

**B. FIELDS SEARCHED**

Minimum documentation searched (classification system followed by classification symbols)

A23J3/16; A23J3/34; A23L15/00; C12N9/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); FSTA/CAplus/AGRICOLA/BIOSIS/MEDLINE/EMBASE (STN); Google

**C. DOCUMENTS CONSIDERED TO BE RELEVANT**

| Category* | Citation of document, with indication, where appropriate, of the relevant passages | Relevant to claim No. |
|---|---|---|
| A | JP 2001-218590 A (AMANO ENZYME INC.) 14 August 2001 (2001-08-14)<br>examples 8-10 | 1-9 |
| A | 山口　庄太郎, 新規酵素の開発技術－プロテイングルタミナーゼの開発と用途開発－,<br>月刊フードケミカル, 2008.12.01, vol.24, no.12, pp.62-65<br>page 63, (2) Application example for soybean protein, fig. 3 | 1-9 |
| A | WO 2020/171106 A1 (AMANO ENZYME INC.) 27 August 2020 (2020-08-27)<br>example 8, fig. 7 | 1-9 |
| A | JP 2015-159765 A (MARUSAN-AI CO., LTD.) 07 September 2015 (2015-09-07)<br>claims, examples | 1-9 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **24 March 2023** | **04 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

International application No.

**PCT/JP2023/002474**

| Patent document cited in search report | | | Publication date (day/month/year) | Patent family member(s) | | | Publication date (day/month/year) |
|---|---|---|---|---|---|---|---|
| JP | 2001-218590 | A | 14 August 2001 | US examples 8-10 EP | 6756221 1106696 | B1 A1 | |
| WO | 2020/171106 | A1 | 27 August 2020 | US example 8, fig. 7 EP | 2022/0151254 3928628 | A1 A1 | |
| JP | 2015-159765 | A | 07 September 2015 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017175976 A
- JP 2000050887 A
- JP 2001218590 A
- WO 2006075772 A

**Non-patent literature cited in the description**

- *Journal of cookery science of Japan*, 1982, vol. 15 (2), 114-118